(19) Europäisches Patentamt
European Patent Office
Office européen des brevets

(11) Veröffentlichungsnummer: **0 373 425 A2**

# (12) EUROPÄISCHE PATENTANMELDUNG

(21) Anmeldenummer: **89122112.9**

(22) Anmeldetag: **30.11.89**

(51) Int. Cl.5: **C07D 237/18, A01N 43/58, C07D 405/12, C07D 409/12, C07D 311/58**

(30) Priorität: **13.12.88 DE 3841850**
**18.07.89 DE 3923659**

(43) Veröffentlichungstag der Anmeldung:
**20.06.90 Patentblatt 90/25**

(84) Benannte Vertragsstaaten:
**BE CH DE ES FR GB IT LI NL**

(71) Anmelder: **BAYER AG**

**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Tietjen, Klaus-Günther, Dr.**
**Am Alten Broich 64a**
**D-4018 Langenfeld(DE)**
Erfinder: **Stendel, Wilhelm, Dr.**
**In den Birken 55**
**D-5600 Wuppertal 1(DE)**
Erfinder: **Wachendorff-Neumann, Ulrike, Dr.**
**Kriescher Strasse 81**
**D-4019 Monheim(DE)**

## (54) Substituierte Pyridazinone, Verfahren zu ihrer Herstellung und ihre Verwendung als Schädlingsbekämpfungsmittel.

(57) Es werden neue substituierte Pyridazinone dar allgemeinen Formel (I)

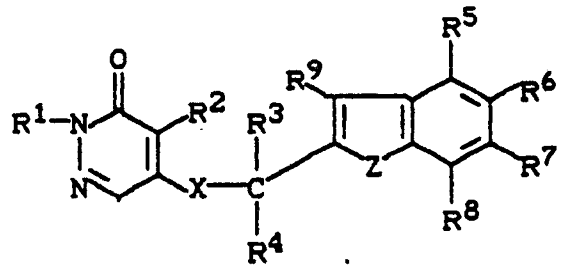

(I)

bereitgestellt, in welcher
$R^1$ für Alkyl, Halogenalkyl, für gegebenenfalls substituiertes Cycloalkyl, für gegebenenfalls substituiertes Cycloalkylalkyl oder für gegebenenfalls substituiertes Aralkyl steht,
$R^2$ für Halogen oder Alkyl steht,
$R^3$ und $R^4$ unabhängig voneinander jeweils für Wasserstoff oder Alkyl stehen,
$R^5$, $R^6$, $R^7$, $R^8$ und $R^9$ unabhängig voneinander jeweils für Wasserstoff, Halogen, Alkyl, Halogenalkyl, Alkoxy, Halogenalkoxy, Alkylthio oder Halogenalkylthio stehen,
X für Sauerstoff oder Schwefel steht und
Z für einen Rest der Formel $-CH=CH-$, $-CH_2-O-$, -O-oder -S- steht.

Die neuen Verbindungen der Formel (I) bestizen eine stark ausgeprägte Wirksamkeit gegen tierische Schädliche, sie weisen insbesondere eine sehr gute insektizide, akarizide und ovizide Wirkung auf.

EP 0 373 425 A2

# Substituierte Pyridazinone, Verfahren zu ihrer Herstellung und ihre Verwendung als Schädlingsbekämpfungsmittel

Die Erfindung betrifft neue substituierte Pyridazinone, mehrere Verfahren zu ihrer Herstellung und ihre Verwendung als Schädlingsbekämpfungsmittel.

Es ist bekannt, daß bestimmte substituierte Pyridazinone, wie beispielsweise die Verbindung 2-t-Butyl-4-chlor-5-{2-[4-(3,3-dimethylbutyl)-2,6-dichlorphenoxy]ethylthio}-pyridazin-(2H)-3-on, die Verbindung 2-t-Butyl-4-chlor-5-(4-t-butyl-phenylmethylthio)-3(2H)-pyridazinon oder die Verbindung 2-t-Butyl-4-chlor-5-[2-(4-methyl-2,6-dichlorphenoxy)-ethylthio]-pyridazin-(2H)-3-on gute Wirksamkeit gegen Schädlinge, insbesondere eine gute insektizide, akarizide, nematizide und fungizide Wirksamkeit besitzen (vgl. z.B. EP 232 825 und EP 134 439).

Die Wirkungshöhe bzw. Wirkungsdauer dieser vorbekannten Verbindungen ist jedoch, insbesondere gegen bestimmte Organismen oder bei niedrigen Anwendungskonzentrationen nicht in allen Anwendungsgebieten völlig zufriedenstellend.

Es wurden neue substituierte Pyridazinone der allgemeinen Formel (I),

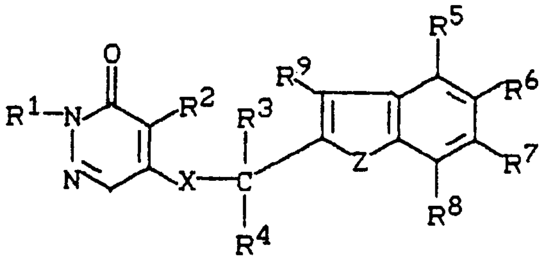

(I)

in welcher

$R^1$ für Alkyl, Halogenalkyl, für gegebenenfalls substituiertes Cycloalkyl, für gegebenenfalls substituiertes Cycloalkylalkyl oder für gegebenenfalls substituiertes Aralkyl steht,

$R^2$ für Halogen oder Alkyl steht,

$R^3$ und $R^4$ unabhängig voneinander jeweils für Wasserstoff oder Alkyl stehen,

$R^5$, $R^6$, $R^7$, $R^8$ und $R^9$ unabhängig voneinander jeweils für Wasserstoff, Halogen, Alkyl, Halogenalkyl, Alkoxy, Halogenalkoxy, Alkylthio oder Halogenalkylthio stehen,

X für Sauerstoff oder Schwefel steht und

Z für einen Rest der Formel $-CH=CH-$, $-CH_2-O-$, -O-oder -S- steht,

gefunden.

Weiterhin wurde gefunden, daß man die neuen substituierten Pyridazinone der allgemeinen Formel (I),

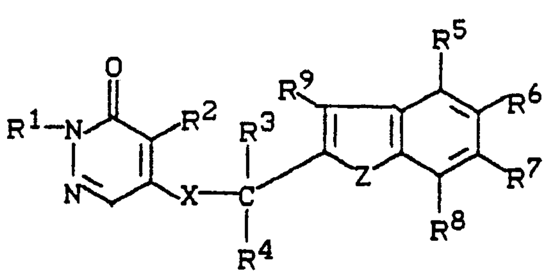

(I)

in welcher

$R^1$ für Alkyl, Halogenalkyl, für gegebenenfalls substituiertes Cycloalkyl, für gegebenenfalls substituiertes Cycloalkylalkyl oder für gegebenenfalls substituiertes Aralkyl steht,

$R^2$ für Halogen oder Alkyl steht,

$R^3$ und $R^4$ unabhängig voneinander jeweils für Wasserstoff oder Alkyl stehen,

$R^5$, $R^6$, $R^7$, $R^8$ und $R^9$ unabhängig voneinander jeweils für Wasserstoff, Halogen, Alkyl, Halogenalkyl, Alkoxy, Halogenalkoxy, Alkylthio oder Halogenalkylthio stehen,

X für Sauerstoff oder Schwefel steht und

Z für einen Rest der Formel -CH = CH-, $-CH_2-O-$, -O-oder -S- steht, erhält, wenn man
(a) 5-Hydroxy- oder 5-Mercaptopyridazinone der Formel (II),

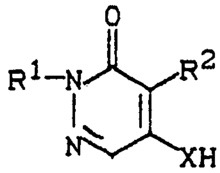

(II)

in welcher
X, $R^1$ und $R^1$ die oben angegebene Bedeutung haben,
mit Alkylierungsmitteln der Formel (III),

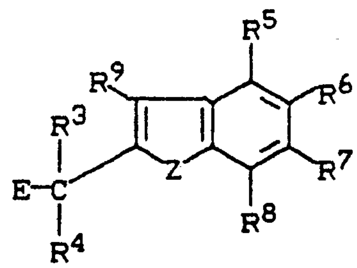

(III)

in welcher
E für eine elektronenanziehende Abgangsgruppe steht und
$R^3$, $R^4$, $R^5$, $R^6$, $R^7$, $R^8$, $R^9$ und Z die oben angegebene Bedeutung haben,
gegebenenfalls in Gegenwart eines Verdünnungsmittels und gegebenenefalls in Gegenwart eines Reaktionshilfsmittels umsetzt oder wenn man
(b) 5-Chlorpyridazinone der Formel (IV),

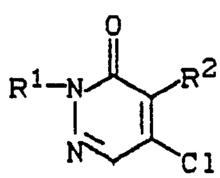

(IV)

in welcher
$R^1$ und $R^2$ die oben angegebene Bedeutung haben,
mit Aralkylalkoholen oder -thiolen der Formel (V),

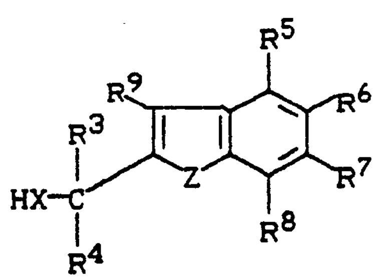

(V)

in welcher
$R^3$, $R^4$, $R^5$, $R^6$, $R^7$, $R^8$, $R^9$, X und Z die oben angegebene Bedeutung haben,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Pyridazinone der allgemeinen Formel (I) gute Wirksamkeit gegen Schädlinge, insbesondere gute insektizide, akarizide und ovizide Wirksamkeit besitzen.

Überraschenderweise zeigen die erfindungsgemäßen substituierten Pyridazinone eine erheblich bessere insektizide Wirksamkeit gegen pflanzenschädigende und warmblüterparasitierende Insekten und Spinnentiere sowie zusätzlich eine bessere ovizide Wirkung, als die aus dem Stand der Technik bekannten substituierten Pyridazinone, wie beispielsweise die Verbindung 2-t-Butyl-4-chlor-5-{2-[4-(3,3-dimethylbutyl)-2,6-dichlorphenoxy]-ethylthio}-pyridazin-(2H)-3-on, die Verbindung 2-t-Butyl-4-chlor-5-(4-t-butyl-phenylmethylthio)-3(2H)-pyridazinon oder die Verbindung 2-t-Butyl-4-chlor-5-[2-(4-methyl-2,6-dichlorphenoxy)-ethylthio]-pyridazin-3(2H)-on, welche chemisch und wirkungsmäßig naheliegende Verbindungen sind.

Die erfindungsgemäßen substituierten Pyridazinone sind durch die Formel (I) allgemein definiert.

Bevorzugt sind Verbindungen der Formel (I), bei welchen

$R^1$ für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils gegebenenfalls einfach oder mehrfach, gleich oder verschieden substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten Alkylteil steht, wobei als Substituenten im Cycloalkylteil jeweils infrage kommen: Alkyl mit 1 bis 4 Kohlenstoffatomen oder Halogen; $R^1$ ferner für gegebenenfalls einfach oder mehrfach, gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^2$ für Fluor, Chlor, Brom, Iod oder für geradkettiges oder verzweigtes Alkyl mit 1 bis 4 Kohlenstoffatomen steht,

$R^3$ und $R^4$ unabhängig voneinander jeweils für Wasserstoff oder für geradkettiges oder verzweigtes Alkyl mit 1 bis 4 Kohlenstoffatomern stehen,

$R^5$, $R^6$, $R^7$, $R^8$ und $R^9$ unabhängig voneinander jeweils für Wasserstoff, Halogen oder für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen stehen,

X für Sauerstoff oder Schwefel steht und

Z für einen Rest der Formel $-CH=CH-$, $-CH_2-O-$ oder -S-steht.

Besonders bevorzugt sind Verbindungen der Formel (I), bei welchen

$R^1$ für Methyl, Ethyl, n- oder i-Propyl, n-, i-, s-oder t-Butyl, für geradkettiges oder verzweigtes Pentyl, für geradkettiges oder verzweigtes Hexyl, für geradkettiges oder verzweigtes Fluoralkyl mit 1 bis 4 Kohlenstoffatomen und 1 bis 5 Fluoratomen, für jeweils gegebenenfalls ein- bis fünffach, gleich oder verschieden durch Fluor, Chlor, Methyl und/oder Ethyl substituiertes Cyclopropyl, Cyclopentyl, Cyclohexyl, Cyclopropylmethyl, Cyclopropylethyl, Cyclopropylpropyl oder Cyclohexylmethyl oder für gegebenenfalls ein- bis dreifach, gleich oder verschieden substituiertes Benzyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy oder Trifluormethylthio,

$R^2$ für Fluor, Chlor, Brom, Methyl, Ethyl, n- oder i-Propyl steht,

$R^3$ und $R^4$ unabhängig voneinander jeweils für Wasserstoff, Methyl oder Ethyl stehen,

$R^5$, $R^6$, $R^7$, $R^8$ und $R^9$ unabhängig voneinander jeweils für Wasserstoff, Fluor, Chlor, Brom, Iod, Methyl, Ethyl, n- oder i-Propyl, n-, i-, s- oder t-Butyl, Trifluormethyl, Trifluormethoxy, Difluormethoxy, Trifluormethylthio, Methoxy, Ethoxy oder Methylthio stehen,

X für Sauerstoff oder Schwefel steht und

Z für einen Rest der Formel $-CH=CH-$, $-CH_2-O-$, -O-oder -S- steht.

Ganz besonders bevorzugt sind Verbindungen der Formel (I), bei welchen

$R^1$ für Methyl, Ethyl, i-Propyl, i-Butyl, t-Butyl, i-Amyl, t-Amyl, Difluor-t-butyl, Trifluor-t-butyl, für Cyclopentyl, Cyclohexyl, für jeweils gegebenenfalls ein- bis fünffach, gleich oder verschieden durch Fluor, Chlor und/oder Methyl substituiertes Cyclopropylmethyl oder Cyclopropylethyl oder für gegebenenfalls einfach oder zweifach, gleich oder verschieden durch Fluor, Chlor, Brom, Methyl, Ethyl, Methoxy, Methylthio, Trifluormethyl, Trifluormethoxy, Difluormethoxy oder Trifluormethylthio substituiertes Benzyl steht,

$R^2$ für Chlor, Brom, Methyl oder Ethyl steht,

$R^3$ und $R^4$ unabhängig voneinander jeweils für Wasserstoff oder Methyl stehen,

$R^5$, $R^6$, $R^7$, $R^8$ und $R^9$ unabhängig voneinander jeweils für Wasserstoff, Fluor, Chlor, Brom, Methyl, Ethyl,

n-oder i-, s- oder t-Butyl, Trifluormethyl, Trifluormethoxy, Difluormethoxy, Trifluormethylthio, Methoxy oder Ethoxy stehen,

X für Sauerstoff oder Schwefel steht und

Z für einen Rest der Formel -CH = CH-, $-CH_2-O-$, -O-oder -S- steht.

Insbesondere bevorzugt sind Verbindungen der Formel (I), bei welchen

$R^1$ für Methyl, Ethyl, i-Propyl, i-Butyl, t-Butyl, i-Amyl, t-Amyl, Difluor-t-butyl, Trifluor-t-butyl, für Cyclopentyl, Cyclohexyl, für jeweils gegebenenfalls ein- bis fünffach, gleich oder verschieden durch Fluor, Chlor und/oder Methyl substituiertes Cyclopropylmethyl oder Cyclopropylethyl oder für gegebenenfalls einfach oder zweifach, gleich oder verschieden durch Fluor, Chlor, Brom, Methyl, Ethyl, Methoxy, Methylthio, Trifluormethyl, Trifluormethoxy, Difluormethoxy oder Trifluormethylthio substituiertes Benzyl steht,

$R^2$ für Chlor, Brom, Methyl oder Ethyl steht,

$R^3$ und $R^4$ unabhängig voneinander jeweils für Wasserstoff oder Methyl stehen,

$R^5$, $R^6$, $R^7$, $R^8$ und $R^9$ unabhängig voneinander jeweils für Wasserstoff, Fluor, Chlor, Brom, Methyl, Ethyl, n-oder i-, s- oder t-Butyl, Trifluormethyl, Trifluormethoxy, Difluormethoxy, Trifluormethylthio, Methoxy oder Ethoxy stehen,

X für Sauerstoff oder Schwefel steht und

Z für einen Rest der Formel $-CH_2-O-$ steht.

Im einzelnen seien außer den bei den Herstellungsbeispielen genannten Verbindungen die folgenden substituierten Pyridazinone der allgemeinen Formel (I) genannt:

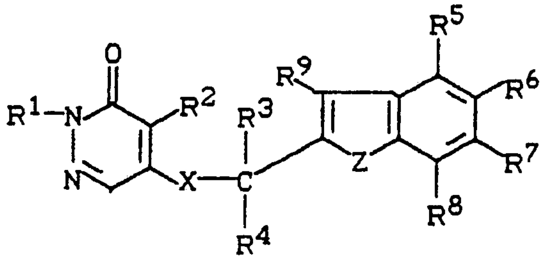

(I)

| $R^1$ | $R^2$ | $R^3$ | $R^4$ | X | 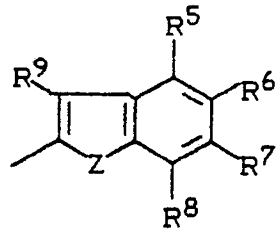 |
|---|---|---|---|---|---|
| $(CH_3)_3C-$ | Cl | H | H | S | 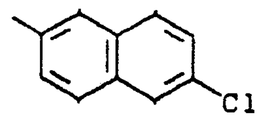 |
| $(CH_3)_3C-$ | Cl | H | H | S | 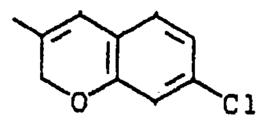 |
| $(CH_3)_3C-$ | Cl | H | H | S | 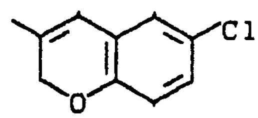 |
| $(CH_3)_3C-$ | Cl | H | H | S | 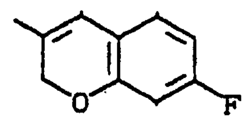 |
| $(CH_3)_3C-$ | Cl | H | H | S | 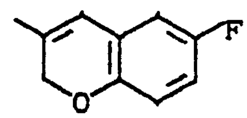 |

| $R^1$ | $R^2$ | $R^3$ | $R^4$ | X | 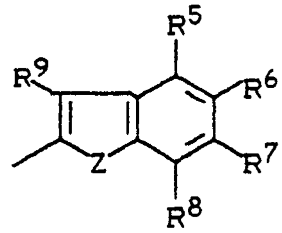 |
|---|---|---|---|---|---|
| 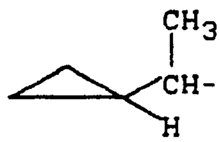 | Cl | H | H | S | 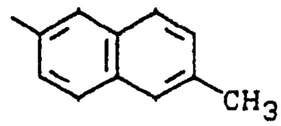 |
| 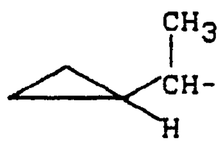 | Cl | H | H | S | 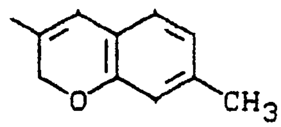 |
| 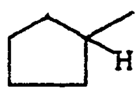 | Cl | H | H | S | 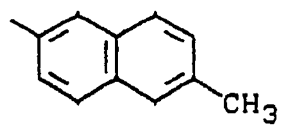 |
| 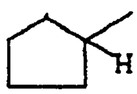 | Cl | H | H | S | 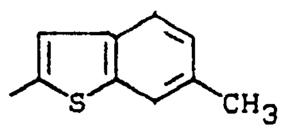 |
| 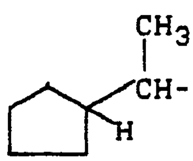 | Cl | H | H | S | 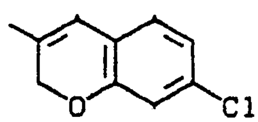 |
| $(CH_3)_3C-$ | Cl | H | H | S | 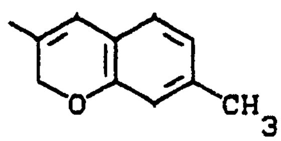 |
| $(CH_3)_3C-$ | Cl | H | H | S | 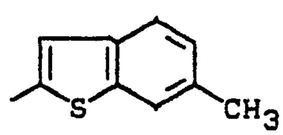 |

| $R^1$ | $R^2$ | $R^3$ | $R^4$ | X | 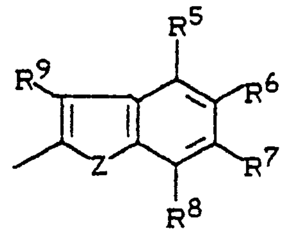 |
|---|---|---|---|---|---|
| $(CH_3)_3C-$ | Cl | H | H | S | 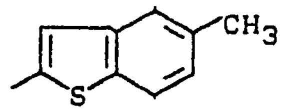 |
| $(CH_3)_3C-$ | Cl | H | H | S | 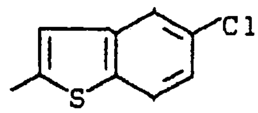 |
| $(CH_3)_3C-$ | Cl | H | H | S | 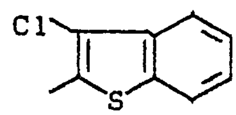 |
| $(CH_3)_3C-$ | Cl | H | H | S | 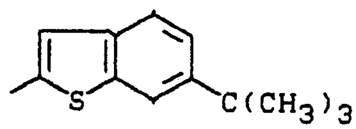 |
| $(CH_3)_3C-$ | Cl | H | H | S | 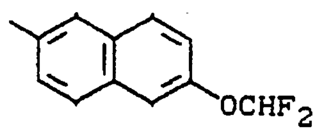 |
| $(CH_3)_3C-$ | Cl | H | H | S | 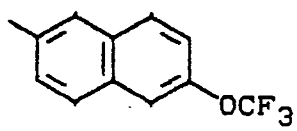 |
| $(CH_3)_3C-$ | Cl | H | H | S | 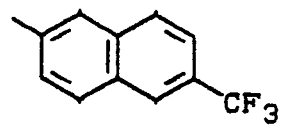 |
| $(CH_3)_3C-$ | Cl | H | H | S | 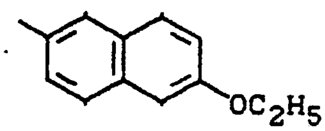 |
| $(CH_3)_3C-$ | Cl | H | H | S | 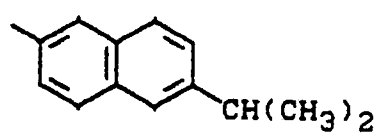 |

| $R^1$ | $R^2$ | $R^3$ | $R^4$ | X | 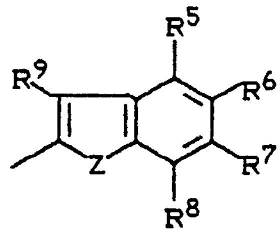 |
|---|---|---|---|---|---|
| $(CH_3)_3C-$ | Cl | H | H | S | 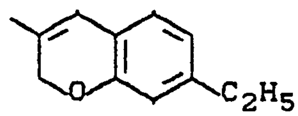 |
| $(CH_3)_3C-$ | Cl | H | H | S | 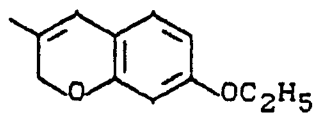 |
| $(CH_3)_3C-$ | Cl | H | H | S | 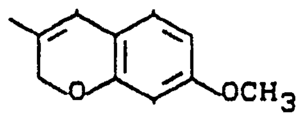 |
| $(CH_3)_3C-$ | Cl | H | H | S | 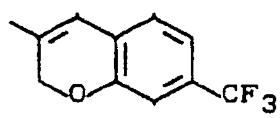 |
| $(CH_3)_3C-$ | Cl | H | H | S | 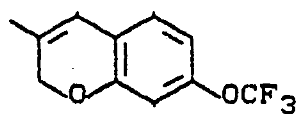 |

Verwendet man beispielsweise 2-t-Butyl-4-chlor-5-hydroxy-pyridazin-3-(2H)-on und 2-Brommethyl-6-methylnaphthalin, als Ausgangsstoffe, so läßt sich der Reaktionsablauf des erfindungsgemäßen Verfahrens (a) durch das folgende Formelschema darstellen:

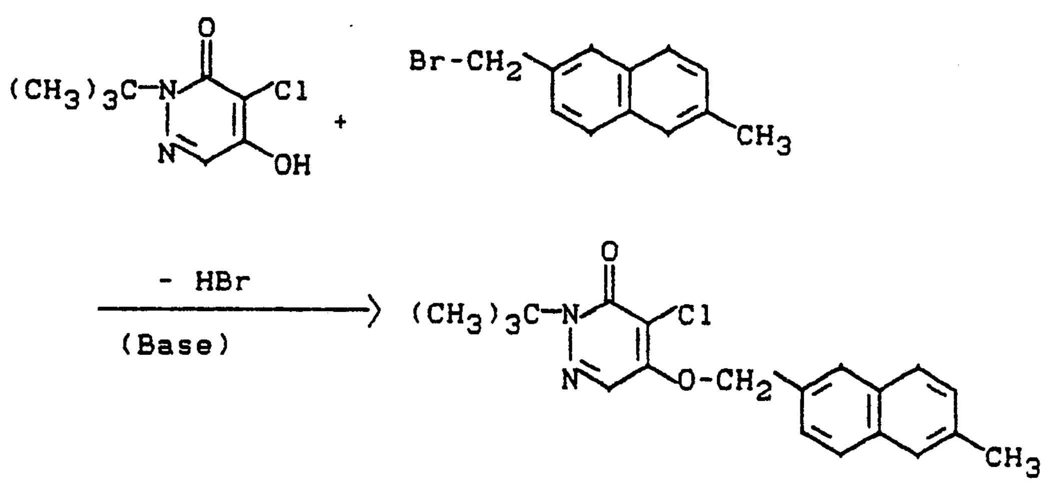


Verwendet man beispielsweise 2-Cyclopropylmethyl-4,5-dichlorpyridazin-3-(2H)-on und 2-Mercaptomethyl-thiophen als Ausgangsstoffe, so läßt sich der Reaktionsablauf des erfindungsgemäßen

Verfahrens (b) durch das folgende Formelschema darstellen:

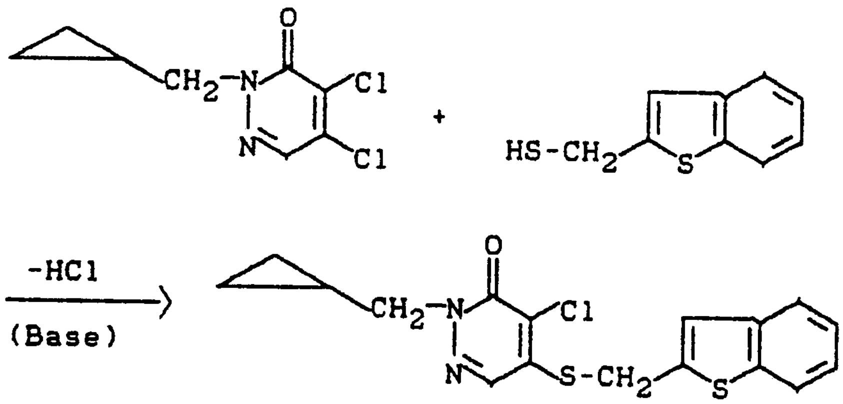


Die zur Durchführung des erfindungsgemäßen Verfahrens (a) als Ausgangsstoffe benötigten 5-Hydroxy- oder 5-Mercaptopyridazinone sind durch die Formel (II) allgemein definiert. In dieser Formel (II) stehen $R^1$, $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Hydroxy- und 5-Mercaptopyridazinone der Formel (II) sind bekannt oder erhältlich in Analogie zu bekannten Verfahren (vgl. z.B. EP 199 281; EP 183 212; Chem. Pharm. Bull. 18, 147-156 [1979]; JP 61/109777; Heterocycles 26, 1 - 4 [1987]; Pestic. Sci. 9, 571 - 581 [1978]; Chem. Zvesti 30, 663 - 673 [1976] bzw. CA 87: 135236y; CS 146172 vom 15.12.1972).

Die zur Durchführung des erfindungsgemäßen Verfahrens (a) weiterhin als Ausgangsstoffe benötigten Aralkylierungsmittel sind durch die Formel (III) allgemein definiert. In dieser Formel (III) stehen $R^3$, $R^4$, $R^5$, $R^6$, $R^7$, $R^8$, $R^9$ und Z vorzugsweise für diejenigen Reste, die bereits im Zusammenhang mit der Beschreibung der erfindungsgemäßen Stoffe der Formel (I) als bevorzugt für diese Substituenten genannt wurden.

E steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (III) sind allgemein bekannte Verbindungen oder erhältlich in Analogie zu allgemein bekannten Verfahren der organischen Chemie (vergl. z.B. US-PS 42 82 227; DE-OS 25 08 335; EP 221 677; Eur. J. med. Chem. 22, 539 - 544 [1987]; US 3 790 600; DE-OS 23 17 106; J. org. Chem. 53, 3634 -3637 [1988]).

Noch nicht bekannt sind die Alkylierungsmittel der Formel (III a)

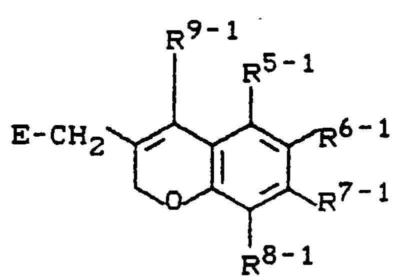

(III a)

in welcher

$R^{5-1}$, $R^{6-1}$, $R^{7-1}$, $R^{8-1}$ und $R^{9-1}$ unabhängig voneinander jeweils für Wasserstoff, für Halogen wie vorzugsweise Fluor, Chlor, Brom, für jeweils geradkettiges oder verzweigtes Alkyl, Alkoxy oder Alkylthio mit jeweils 1 bis 4 Kohlenstoffatomen wie vorzugsweise Methyl, Ethyl, n-, i-, s- oder t-Butyl, Methoxy, Ethoxy oder Methylthio sowie für jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen, wie vorzugsweise Trifluormethyl, Trifluormethoxy, Difluormethoxy oder Trifluormethylthio stehen, wobei jedoch nicht gleichzeitig alle Substituenten für Wasserstoff stehen, und

E für Halogen, wie vorzugsweise Chlor, Brom oder Iod sowie für jeweils gegebenenfalls substituiertes

Alkylsulfonyloxy, Alkoxysulfonyloxy oder Arylsulfonyloxy wie vorzugsweise Methansulfonyloxy, Trifluormethansulfonyloxy, Methoxysulfonyloxy, Ethoxysulfonyloxy oder p-Toluolsulfonyloxy steht.

Die Alkylierungsmitel der Formel (III a) werden in Analogie zu allgemein bekannten Verfahren erhalten, indem man entsprechende Alkohole der Formel (V a)

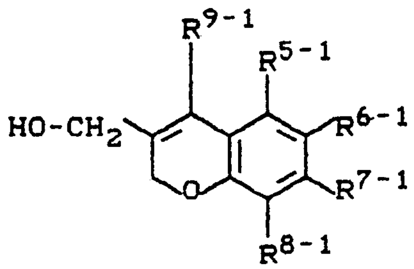

(V a)

in welcher
$R^{5-1}$, $R^{6-1}$, $R^{7-1}$, $R^{8-1}$ und $R^{9-1}$ unabhängig voneinander jeweils für Wasserstoff, für Halogen wie vorzugsweise Fluor, Chlor, Brom, für jeweils geradkettiges oder verzweigtes Alkyl, Alkoxy oder Alkylthio mit jeweils 1 bis 4 Kohlenstoffatomen wie vorzugsweise Methyl, Ethyl, n-, i-, s- oder t-Butyl, Methoxy, Ethoxy oder Methylthio sowie für jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen, wie vorzugsweise Trifluormethyl, Trifluormethoxy, Difluormethoxy oder Trifluormethylthio stehen, wobei jedoch nicht gleichzeitig alle Substituenten für Wasserstoff stehen,
mit entsprechenden Halogenierungs- bzw. Sulfonylierungsmitteln umsetzt (vgl. auch die Herstellungsbeispiele).

Die Alkohole der Formel (V a) sind ebenfalls noch nicht bekannt. Auch sie werden in Analogie zu allgemein bekannten Verfahren erhalten, indem man entsprechende 2(H)-Chromen-3-carbonsäuren der Formel (VI)

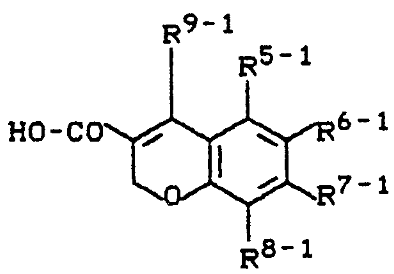

(VI)

in welcher
$R^{5-1}$, $R^{6-1}$, $R^{7-1}$, $R^{8-1}$ und $R^{9-1}$ die oben angegebenen Bedeutungen haben,
oder entsprechende 2(H)-Chromen-3-carbaldehyde der Formel (VII)

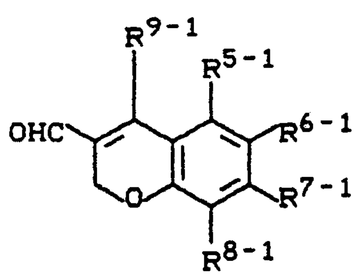

(VII)

in welcher
$R^{5-1}$, $R^{6-1}$, $R^{7-1}$, $R^{8-1}$ und $R^{9-1}$ die oben angegebenen Bedeutungen haben,
jeweils reduziert, vorzugsweise mittels Natriumborhydrid oder Lithiumaluminiumhydrid (vgl. auch die Herstellungsbeispiele).

Die 2(H)-Chromen-3-carbonsäuren der Formel (VI) sowie die 2(H)-Chromen-3-carbaldehyde der Formel (VII) sind bekannt (vgl. z.B. J. Org. Chem. 39, 2426 (1974) oder Eur. J. med. Chem. - Chim. Ther., 10(1), 72 - 8) bzw. können in analoger Weise hergestellt werden.

Die zur Durchführung des erfindungsgemäßen Verfahrens (b) als Ausgangsstoffe benötigten 5-Chlorpyridazinone sind durch die Formel (IV) allgemein definiert. In dieser Formel (IV) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Chlorpyridazinone der Formel (IV) sind bekannt oder erhältlich in Analogie zu bekannten Verfahren (vergl. z.B. EP 169 375; Chem. Zvesti 38, 239 - 246 [1984] bzw. CA 101: 110848u; GB 2095 669; Synthesis 1981, 631 - 633.)

Die zur Durchführung des erfindungsgemäßen Verfahrens (b) weiterhin als Ausgangsstoffe benötigten Aralkylalkohole und -thiole sind durch die Formel (V) allgemein definiert. In dieser Formel (V) stehen $R^3$, $R^4$, $R^5$, $R^6$, $R^7$, $R^8$, $R^9$, X und Z vorzugsweise für diejenigen Reste, die bereits im Zusammenhang mit der Beschreibung der erfindungsgemäßen Stoffe der Formel (I) als bevorzugt für diese Substituenten genannt wurden.

Die Aralkylalkohole und -thiole der Formel (V) sind allgemein bekannte Verbindungen oder erhältlich in Analogie zu allgemein bekannten Verfahren der organischen Chemie (vergl. z.B. DE-OS 23 17 106; US-PS 37 90 600; J. Am. Chem. Soc. 106, 1779 - 1789 [1984]; Synthesis 1987, 647 - 648; Jp 62/87529; Eur. J. Med. Chem. 22, 539 - 544 [1987]; J. Heterocycl. Chem. 23, 1211 - 1214 [1986]; J. Chem. Soc. Perkin Trans. 1, 1972, 787 - 792).

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die erfindungsgemäßen Verfahren (a) und (b) werden vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (a) setzt man pro Mol an 5-Hydroxy- oder 5-Mercaptopyridazinon der Formel (II) im allgemeinen 1,0 bis 5,0 Mol, vorzugsweise 1,0 bis 3,0 Mol an Aralkylierungsmittel der Formel (III) und gegebenenfalls 1,0 bis 5,0 Mol, vorzugsweise 1,0 bis 3,0 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (b) setzt man pro Mol an 5-Chlorpyridazinon der Formel (IV) im allgemeinen 1,0 bis 5,0 Mol, vorzugsweise 1,0 bis 3,0 Mol an Aralkylalkohol oder -thiol der Formel (V) und gegebenenfalls 1,0 bis 5,0 Mol, vorzugsweise 1,0 bis 3,0 Mol an Reaktionshilfsmittel ein.
Die Reaktionsdurchführung, Aufarbeitung und Isolierung der Reaktionsprodukte erfolgt nach allgemein üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in

Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp., Ctenocephalides spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensilble und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide, akarizide und ovizide Wirksamkeit aus.
Sie lassen sich mit besonders gutem Erfolg zur Bekämpfung von pflanzenschädigenden Insekten, wie beispielsweise gegen die grüne Pfirsichblattlaus (Myzus persicae) oder gegen die Larven des Meerettichblattkäfers (Phaedon cochleariae) oder gegen die Larven der Kohlschabe (Plutella xylostella) oder gegen die Larven der grünen Reiszikade (Nephotettix cincticeps) oder gegen die Larven des Baumwollkapselwurms (Heliothis armigera) oder gegen die Larven des Heerwurms (Spodoptera frugiperda); zur Bekämpfung von pflanzenschädigenden Milben, wie beispielsweise gegen die gemeine Spinnmilbe oder die Bohnenspinnmilbe (Tetranychus urticae) sowie als Ovizide, beispielsweise gegen die Eier des Baumwollkapselwurms (Heliothis armigera) eingesetzen. Außerdem zeigen die neuen Wirkstoffe gute entwicklungshemmende Wirkung beispielsweise bei der Mittelmeerfruchtfliege (Ceratitis capitata).

Darüberhinaus lassen sie sich mit besonders gutem Erfolg zur Bekämpfung von parasitisch lebenden Warmblüterschädlingen, wie beispielsweise gegen die Larven der Goldfliege (Lucilia cuprina), gegen Rinderzecken (Boophilus microplus) oder gegen Räudemilben (Psoroptes ovis) einsetzen.

In entsprechenden Aufwandmengen zeigen die erfindungsgemäßen Wirkstoffe auch gute fungizide Wirkung, wie insbesondere gegen Pyricularia oryzae am Reis.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüll massen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte ali phatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die Biologische Wirksamkeit der erfindungsgemäßen Verbindungen soll anhand der folgenden Beispiele erläutert werden.

Herstellungsbeispiele:

Beispiel 1:

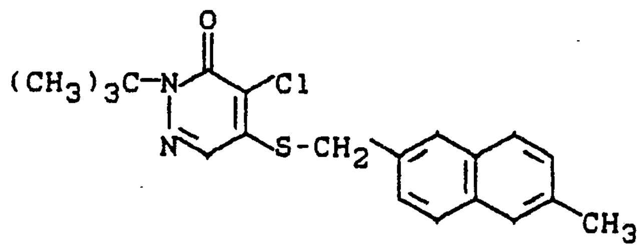

(Verfahren a)

Zu 8 g (0,03 Mol) 2-t-Butyl-4-chlor-5-mercapto-3-pyridazin-(2H)-on (vergl. z.B. JP 61/109 777) und 6,9 g (0,05 Mol) Kaliumcarbonat in 50 ml Dimethylformamid gibt man 9,45 g (0,04 Mol) 2-Brommethyl-6-methylnaphthalin (vergl. z.B. Tetrahedron 34, 769 - 777 [1978]) und rührt 16 Stunden bei Raumtemperatur. Zur Aufarbeitung wird mit 250 ml Wasser verdünnt, zweimal mit jeweils 80 ml Dichlormethan extrahiert, die vereinigten organischen Phasen über Natriumsulfat getrocknet, im Vakuum eingeengt und durch Chromatographie an Kieselgel (Dichlormethan) gereinigt.

Man erhält 8,6 g (77 % der Theorie) an 2-t-Butyl-4-chlor-5-[(6-methyl-2-naphthyl)-methylthio]-3(2H)-pyridazinon vom Schmelzpunkt 107 °C.

Beispiel 2

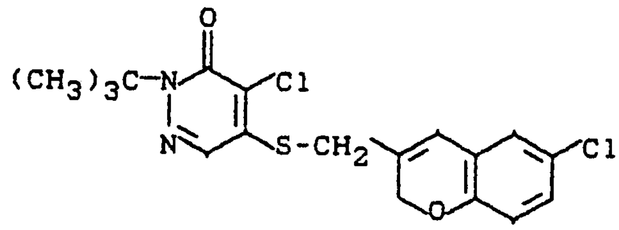

(Verfahren a)

Zu einer Lösung von 6,6 g (0,03 mol) 2-t-Butyl-4-chlor-5-mercapto-3-pyridazin-(2H)-on und 7,3 g (0,053 mol) Kaliumcarbonat in 50 ml Dimethylformamid gibt man unter leichter Kühlung 9,2 g (0,042 mol) 6-Chlor-3-chlormethyl-2(H)-chromen und rührt 16 Stunden bei Raumtemperatur. Zur Aufarbeitung wird mit 250 ml Wasser verdünnt, zweimal mit jeweils 80 ml Dichlormethan extrahiert, die vereinigten organischen Phasen über Natriumsulfat getrocknet, im Vakuum eingeengt und durch Chromatographie an Kieselgel (Dichlormethan) gereinigt.

Man erhält 8,2 g (68 % der Theorie) an 2-t-Butyl-4-chlor-5-[(6-chlor-2(H)-chromen-3-yl)-methylthio]-3-(2H)-pyridazinon vom Schmelzpunkt 82 °C.

Herstellung des Ausgangsproduktes

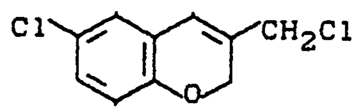

Zu einer Lösung von 8,8 g (0,045 mol) 3-Hydroymethyl-6-chlor-2(H)-chromen in 50 ml Methylenchlorid tropft man 8,5 g (0,07 mol) Thionylchlorid und erhitzt 16 Stunden unte Rückfluß. Nach dem Abkühlen wird die Reaktionsmischung in 100 ml Wasser eingegossen, die organische Phase abgetrennt und zweimal mit Wasser gewaschen, mit Natriumsulfat getrocknet und eingedampft. Man erhält 9,2 g (95 % der Theorie) an 3-Chlormethyl-6-chlor-2(H)-chromen.
$^1$H-NMR ($CDCl_3$) $\delta$ (ppm): 4,15 (s, 2H), 4,83 (s, 2H), 6,4 (s, 1H)

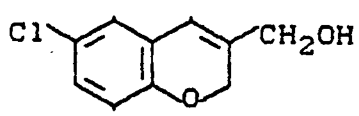

Zu einer Lösung von 20 g (0,095 mol) 6-Chlor-2(H)-chromen-3-carbonsäure in 150 ml Tetrahydrofuran gibt man portionsweise 2,7 g (0,071 mol) Lithiumaluminiumhydrid und rührt anschließend 16 Stunden bei Raumtemperatur. Zur Hydrolyse wird unter Eiskühlung mit einer Mischung aus 8 ml Essigester und 3 ml Wasser versetzt und anschließend bei 50 °C mit 5%iger Natronlauge, bis ein filtrierbarer, grauer Niederschlag entstanden ist, von dem abgesaugt wird. Das Filtrat wird eingedampft, der Rückstand mit Ether/Wasser aufgenommen, die organische Phase über Natriumsulfat getrocknet und eingedampft. Man erhält 8,8 g (47 % der Theorie) an 3-Hydroxymethyl-6-chlor-2(H)-chromen.
(e/z) = 196/198 (30 % / 10 %), 165/167 (100 % / 70 %)

Beispiel 3

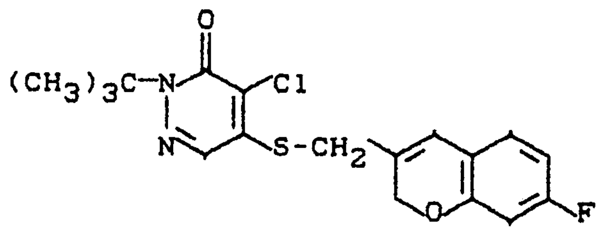

(Verfahren a)

Gemäß Beispiel 2 erhält man 2-t-Butyl-4-chlor-5-[(7-fluor-2(H)-chromen-3-yl)-methylthio]-3(2H)-pyridazinon
$^1$H-NMR ($CDCl_3$) δ (ppm): 4,81 (s, 2H), 3,25 (s, 2H), 6,49 (s, 1H), 7,65 (s, 1H)

Herstellung des Ausgangsproduktes

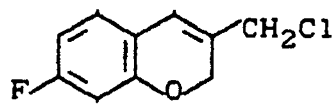

Gemäß Beispiel 2 erhält man 3-Chlormethyl-7-fluor-2(H)-chromen.
$^1$H-NMR ($CDCl_3$) δ (ppm): 4,15 (s, 2H), 4,85 (s, 2H), 6,45 (s, 1H)

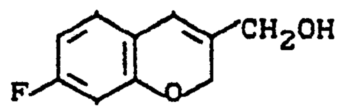

Zu einer Lösung von 36 g (0,2 mol) 7-Fluor-2(H)-chromen-3-carbaldehyd in 250 ml Methanol gibt man portionsweise unter Eiskühlung bei 10 - 20° C 27,1 g (0,71 mol) Natriumborhydrid und rührt 16 Stunden bei Raumtemperatur. Die Reaktionsmischung wird mit 2 N HCl auf pH 7 - 8 gestellt und eingeengt. Der Rückstand wird in Methylenchlorid aufgenommen, mit Wasser gewaschen, die organische Phase getrocknet und eingedampft. Man erhält 21 g 58 % der Theorie) an 3-Hydroxymethyl-7-fluor-2(H)-chromen.
$^1$H-NMR ($CDCl_3$) δ (ppm): 4,18 (s, 2H), 4,8 (s, 2H), 1,85 ( , 1H)

In entsprechender Weise und gemäß den allgemeinen Angaben zur Herstellung erhält man die folgenden substituierten Pyridazinone der allgemeinen Pyridazinone oder allgemeinen Formel (I):

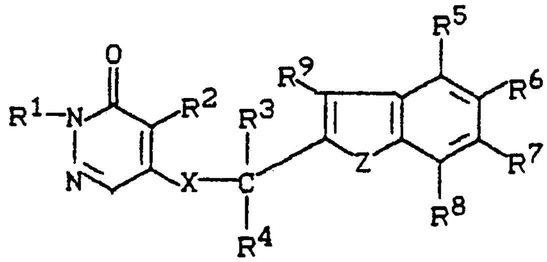

(I)

| Bsp. Nr. | $R^1$ | $R^2$ | $R^3$ | $R^4$ | X | 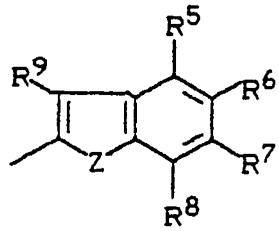 | physikal. Eigenschaften |
|---|---|---|---|---|---|---|---|
| 4 | $(CH_3)_3C-$ | Cl | $CH_3$ | H | S | 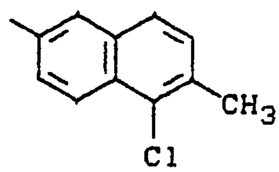 | Fp: 162° C |
| 5 | $(CH_3)_3C-$ | Cl | H | H | S | 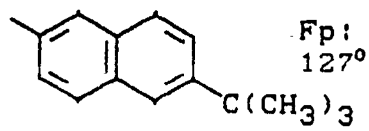 | Fp: 127° C |
| 6 | $(CH_3)_3C-$ | Cl | H | H | S | 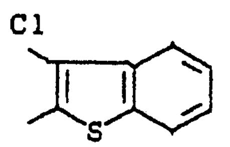 | Fp: 164° C |
| 7 | $(CH_3)_3C-$ | Cl | H | H | S | 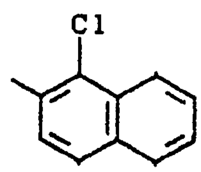 | Fp: 159° C |
| 8 | $(CH_3)_3C-$ | Cl | H | H | S | 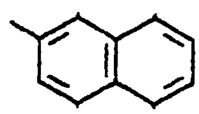 | Fp: 134° C |

| Bsp. Nr. | $R^1$ | $R^2$ | $R^3$ | $R^4$ | X | 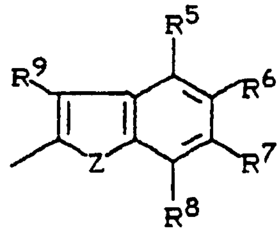 | physikal. Eigen-schaften |
|---|---|---|---|---|---|---|---|
| 9 | $(CH_3)_3C-$ | Cl | H | H | S | 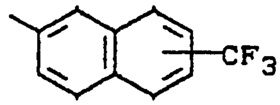 | Fp: 103-104° C |
| 10 | $(CH_3)_3C-$ | Cl | H | H | S | 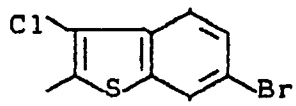 | Fp: 87-88° C |
| 11 | $(CH_3)_3C-$ | Cl | H | H | S | 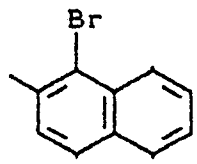 | Fp: 169° C |
| 12 | $(CH_3)_3C-$ | Cl | H | H | S | 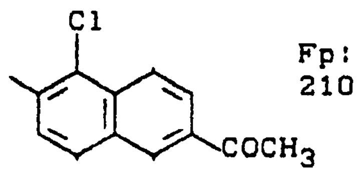 | Fp: 210-211° C |
| 13 | $(CH_3)_3C-$ | Cl | H | H | S | 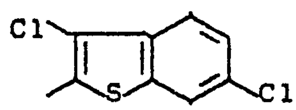 | Fp: 154° C |
| 14 | $(CH_3)_3C-$ | Cl | H | H | S | 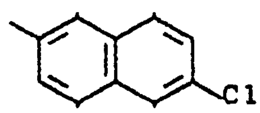 | Fp: 64-65° C |
| 15 | $(CH_3)_3C-$ | Cl | H | H | S | 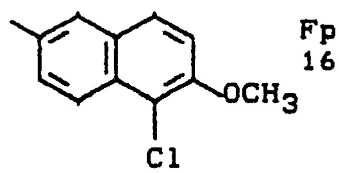 | Fp: 167° C |
| 16 | $(CH_3)_3C-$ | Cl | H | H | S | 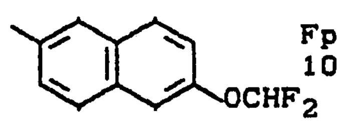 | Fp: 102° C |

Anwendungsbeispiele

In den folgenden Anwendungsbeispielen wurden die nachstehend aufgeführten Verbindungen als Vergleichsubstanzen eingesetzt:

(A)

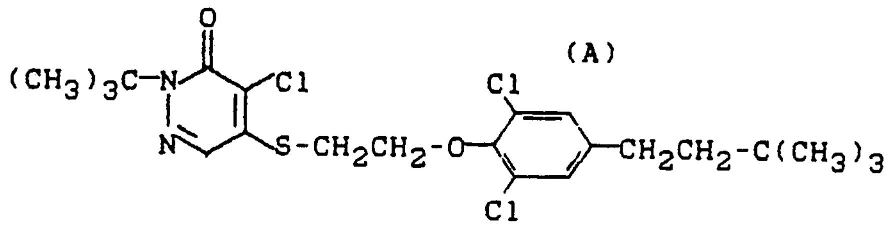

2-t-Butyl-4-chlor-5-{2-[4-(3,3-dimethylbutyl)-2,6-dichlorphenoxy]-ethylthio}-3(2H)-pyridazinon
(bekannt aus EP 232 825)

(B)

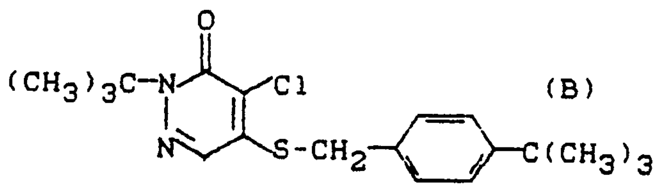

2-t-Butyl-4-chlor-5-(4-t-butyl-phenylmethylthio)-3(2H)-pyridazinon
(bekannt aus EP 134 439)

(C)

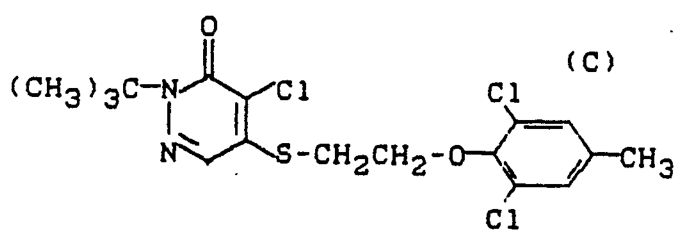

2-t-Butyl-4-chlor-5-[2-(4-methyl-2,6-dichlorphenoxy)-ethylthio]-3(2H)-pyridazinon
(bekannt aus EP 232 825)

Beispiel A

Test mit Boophilus microplus resistent/OP-resistenter Biarra-Stamm

Lösungsmittel: 35 Gewichtsteile Ethylenglykolmonomethylether

35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigt z.B die Verbindung 1 der Herstellungsbeispiele überlegene Wirkung gegenüber dem Stand der Technik.

Beispiel B

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigt z.B. die Verbindung 1 der Herstellungsbeispiele überlegene Wirkung gegenüber dem Stand der Technik.

Beispiel C

Test mit Psoroptes ovis

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

Etwa 10 - 25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigt z.B. die Verbindung 1 der Herstellungsbeispiele überlegene Wirkung gegenüber dem Stand der Technik.

Beispiel D

Phaedon-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden mit der Wirkstoffzubereitung der gewünschten Konzentration behandelt. Ein Blatt der behandelten Pflanze wird in eine Plastikdose gelegt und mit Larven ($L_3$) des Meerrettichkäfers (Phaedon cochleariae) besetzt. Nach 2 bis 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigt z.B. die Verbindung 1 der Herstellungsbeispiele überlegene Wirkung gegenüber dem Stand der Technik.

Beispiel E

Plutella-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden mit der Wirkstoffzubereitung der gewünschten Konzentration behandelt. Ein Blatt der behandelten Pflanze wird in eine Plastikdaose gelegt und mit Larven ($L_2$) der Kohlschabe (Plutella xylostella) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigt z.B. die Verbindung 1 der Herstellungsbeispiele überlegene Wirkung gegenüber dem Stand der Technik.

Beispiel F

Spodoptera-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojabohnenpflanzen (Glycine soja) werden mit der Wirkstoffzubereitung der gewünschten Konzentration behandelt. In zehnfacher Wiederholung wird je ein Blatt der behandelten Pflanze in eine Plastikdose gelegt und mit je einer Larve ($L_2$) des Heerwurms (Spodoptera frugiperda) besetzt. Nach 3 Tagen füttert man je Dose mit einem weiteren Blatt der entsprechenden Pflanze nach. Am 7. Tag werden die Larven auf unbehandeltes Kunstfutter umgesetzt.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung 1 der Herstellungsbeispiele überlegene Wirkung gegenüber dem Stand der Technik.

Beispiel G

Heliothis - Test

Losungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine soja) werden mit der Wirkstoffzubereitung der gewünschten Konzentration behandelt. In zehnfacher Wiederholung wird je ein Blatt der behandelten Pflanze in eine Plastikdose gelegt und mit je einer Larve ($L_2$) des Baumwollkapselwurms (Heliothis armigera) besetzt. Nach 3 Tagen füttert man je Dose mit einem weiteren Blatt der entsprechenden Pflanze nach Am 7. Tag werden die Larven auf unbehandeltes Kunstfutter umgesetzt.

Nach der gewünschten Zeit wird die Abtötung in % bestimmt. Dabei bedeutet 100 %, dar alle Tiere abgetötet wurden; 0 % bedeutet, daß keine Tiere abgetötet wurden.

Bei diesem Test zeigt z.B. die Verbindung 1 der Herstellungsbeispiele überlegene Wirkung gegenüber dem Stand der Technik.

Beispiel H

Entwicklungshemmtest mit Ceratitis capitata (Mittelmeerfruchtfliege)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Je 20 Eier der Mittelmeerfruchtfliege werden auf Kunstfutterbrei in ein Döschen gelegt. Das Futter ist mit Wirkstoff in der angegebenen Konzentration behandelt. Die Summe der abgetöteten Eier, Larven, Puppen und Imagines bezogen auf die Zahl eingesetzter Eier ergibt die Abtötung in %.

Dabei bedeutet 100 %, daß alle Tiere abgetötet wurden; 0 % bedeutet, daß keine Tiere abgetötet wurden.

Bei diesem Test zeigt z.B. die Verbindung 1 der Herstellungsbeispiele überlegene Wirkung gegenüber dem Stand der Technik.

Beispiel I

Nephotettix-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der grünen Reiszikade (Nephotettix cincticeps) besetzt.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigt z.B. die Verbindung 1 der Herstellungsbeispiele überlegene Wirkung gegenüber dem Stand der Technik.

Beispiel J

Myzus-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind,

werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigt z.B. die Verbindung 1 der Herstellungsbeispiele überlegene Wirkung gegenüber dem Stand der Technik.

Beispiel K

Ovizide Wirkung auf Eigelege von Heliothis armigera (Baumwollkapselwurm)

Lösungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

In die Wirkstoffzubereitungen der gewünschten Konzentration wurden 2 Tage alte Eigelege auf Filterpapier für 30 Sekunden eingetaucht und für 6 Tage im Labor unter Langtagbedingungen in geschlossenen Petrischalen deponiert. Kriterium für die Wirkungsbeurteilung war die prozentuale Schlupfverhinderung im Vergleich zu unbehandelten Eigelegen.

Bei diesem Test zeigt z.B. die Verbindung 1 der Herstellungsbeispiele überlegene Wirkung gegenüber dem Stand der Technik.

Beispiel L

Tetranychus-Test (OP-resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration tropfnaß bespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die Verbindung 1 der Herstellungsbeispiele überlegene Wirkung gegenüber dem Stand der Technik.

## Ansprüche

1. Substituierte Pyridazinone der allgemeinen Formel (I),

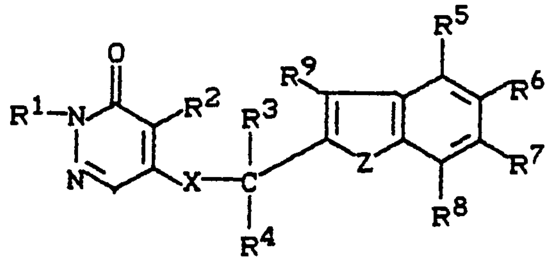

(I)

in welcher
$R^1$ für Alkyl, Halogenalkyl, für gegebenenfalls substituiertes Cycloalkyl, für gegebenenfalls substituiertes Cycloalkylalkyl oder für gegebenenfalls substituiertes Aralkyl steht,
$R^2$ für Halogen oder Alkyl steht,
$R^3$ und $R^4$ unabhängig voneinander jeweils für Wasserstoff oder Alkyl stehen,
$R^5$, $R^6$, $R^7$, $R^8$ und $R^9$ unabhängig voneinander jeweils für Wasserstoff, Halogen, Alkyl, Halogenalkyl, Alkoxy, Halogenalkoxy, Alkylthio oder Halogenalkylthio stehen,
X für Sauerstoff oder Schwefel steht und
Z für einen Rest der Formel $-CH=CH-$, $-CH_2-O-$, -O-oder -S- steht.

2. Substituierte Pyridazinone der allgemeinen Formel (I) gemäß Anspruch 1, in welcher
$R^1$ für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils gegebenenfalls einfach oder mehrfach, gleich oder verschieden substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten Alkylteil steht, wobei als Substituenten im Cycloalkylteil jeweils infrage kommen: Alkyl mit 1 bis 4 Kohlenstoffatomen oder Halogen;
$R^1$ ferner für gegebenenfalls einfach oder mehrfach, gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
$R^2$ für Fluor, Chlor, Brom, Iod oder für geradkettiges oder verzweigtes Alkyl mit 1 bis 4 Kohlenstoffatomen steht,
$R^3$ und $R^4$ unabhängig voneinander jeweils für Wasserstoff oder für geradkettiges oder verzweigtes Alkyl mit 1 bis 4 Kohlenstoffatomern stehen,
$R^5$, $R^6$, $R^7$, $R^8$ und $R^9$ unabhängig voneinander jeweils für Wasserstoff, Halogen oder für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen stehen,
X für Sauerstoff oder Schwefel steht und
Z für einen Rest der Formel $-CH=CH-$, $-CH_2-O-$, -O- oder -S- steht.

3. Substituierte Pyridazinone der allgemeinen Formel (I) gemäß Anspruch 1, dadurch gekennzeichnet, daß
$R^1$ für Methyl, Ethyl, n- oder i-Propyl, n-, i-, s-oder t-Butyl, für geradkettiges oder verzweigtes Pentyl, für geradkettiges oder ver zweigtes Hexyl, für geradkettiges oder verzweigtes Fluoralkyl mit 1 bis 4 Kohlenstoffatomen und 1 bis 5 Fluoratomen, für jeweils gegebenenfalls ein- bis fünffach, gleich oder verschieden durch Fluor, Chlor, Methyl und/oder Ethyl substituiertes Cyclopropyl, Cyclopentyl, Cyclohexyl, Cyclopropylmethyl, Cyclopropylethyl, Cyclopropylpropyl oder Cyclohexylmethyl oder für gegebenenfalls einbis dreifach, gleich oder verschieden substituiertes Benzyl steht,
wobei als Substituenten infrage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Trifluormethyl, Trifluormethoxy, Difluormethoxy oder Trifluormethylthio,
$R^2$ für Fluor, Chlor, Brom, Methyl, Ethyl, n- oder i-Propyl steht,
$R^3$ und $R^4$ unabhängig voneinander jeweils für Wasserstoff, Methyl oder Ethyl stehen,
$R^5$, $R^6$, $R^7$, $R^8$ und $R^9$ unabhängig voneinander jeweils für Wasserstoff, Fluor, Chlor, Brom, Iod, Methyl, Ethyl, n- oder i-Propyl, n-, i-, s- oder t-Butyl, Trifluormethyl, Trifluormethoxy, Difluormethoxy, Trifluormethylthio, Methoxy, Ethoxy oder Methylthio stehen,
X für Sauerstoff oder Schwefel steht und
Z für einen Rest der Formel $-CH=CH-$, $-CH_2-O-$, -O- oder -S- steht.

4. Substituierte Pyridazinone der allgemeinen Formel (I) gemäß Anspruch 1, dadurch gekennzeichnet, daß
$R^1$ für Methyl, Ethyl, i-Propyl, i-Butyl, t-Butyl, i-Amyl, t-Amyl, Difluor-t-butyl, Trifluor-t-butyl, für Cyclopentyl, Cyclohexyl, für jeweils gegebenenfalls ein- bis fünffach, gleich oder verschieden durch Fluor, Chlor und/oder Methyl substituiertes Cyclopropylmethyl oder Cyclopropylethyl oder für gegebenenfalls einfach oder zweifach, gleich oder verschieden durch Fluor, Chlor, Brom, Methyl, Ethyl, Methoxy, Methylthio, Trifluormethyl, Trifluormethoxy, Difluormethoxy oder Trifluormethylthio substituiertes Benzyl steht,
$R^2$ für Chlor, Brom, Methyl oder Ethyl steht,

$R^3$ und $R^4$ unabhängig voneinander jeweils für Wasserstoff oder Methyl stehen,
$R^5$, $R^6$, $R^7$, $R^8$ und $R^9$ unabhängig voneinander jeweils für Wasserstoff, Fluor, Chlor, Brom, Methyl, Ethyl, Trifluormethoxy, n-, i-, s-oder t-Butyl, Trifluormethyl, Difluormethoxy, Trifluormethylthio, Methoxy oder Ethoxy stehen,
X für Sauerstoff oder Schwefel steht und
Z für einen Rest der Formel -CH=CH-, $-CH_2-O-$, -O- oder -S- steht.

5. Verfahren zur Herstellung von substituierten Pyridazinonen der allgemeinen Formel (I),

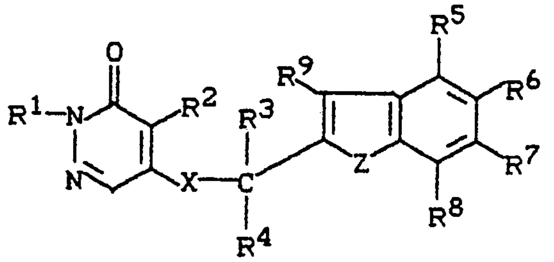

(I)

in welcher
$R^1$ für Alkyl, Halogenalkyl, für gegebenenfalls substituiertes Cycloalkyl, für gegebenenfalls substituiertes Cycloalkylalkyl oder für gegebenenfalls substituiertes Aralkyl steht,
$R^2$ für Halogen oder Alkyl steht,
$R^3$ und $R^4$ unabhängig voneinander jeweils für Wasserstoff oder Alkyl stehen,
$R^5$, $R^6$, $R^7$, $R^8$ und $R^9$ unabhängig voneinander jeweils für Wasserstoff, Halogen, Alkyl, Halogenalkyl, Alkoxy, Halogenalkoxy, Alkylthio oder Halogenalkylthio stehen,
X für Sauerstoff oder Schwefel steht und
Z für einen Rest der Formel -CH=CH-, $-CH_2-O-$, -O- oder -S- steht,
dadurch gekennzeichnet, daß man entweder
(a) 5-Hydroxy- oder 5-Mercaptopyridazinone der Formel (II),

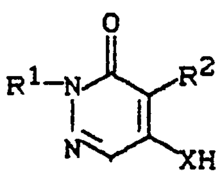

(II)

in welcher
X, $R^1$ und $R^2$ die oben angegebene Bedeutung haben,
mit Alkylierungsmitteln der Formel (III),

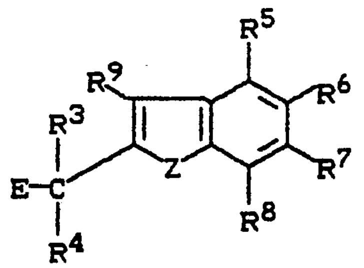

(III)

in welcher
E für eine elektronenanziehende Abgangsgruppe steht und
$R^3$, $R^4$, $R^5$, $R^6$, $R^7$, $R^8$, $R^9$ und Z die oben angegebene Bedeutung haben,
gegebenenfalls in Gegenwart eines Verdünnungsmittels und gegebenenefalls in Gegenwart eines Reaktionshilfsmittels umsetzt
oder daß man

(b) 5-Chlorpyridazinone der Formel (IV),

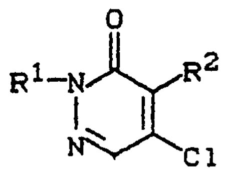

(IV)

in welcher
$R^1$ und $R^2$ die oben angegebene Bedeutung haben,
mit Aralkylalkoholen oder -thiolen der Formel (V),

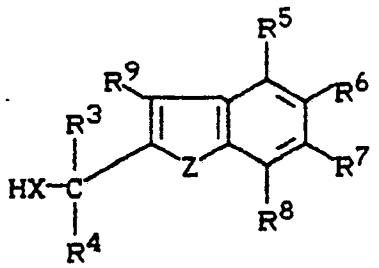

(V)

in welcher
$R^3$, $R^4$, $R^5$, $R^6$, $R^7$, $R^8$, $R^9$, X und Z die oben angegebene Bedeutung haben,
gegebenenfalls in Gegenwart eines Verdünnungsmittels und gegebenenfalls in Gegenwart eines Reaktionshilfsmittels umsetzt.

6. Schädlingsbelämpfungsmittel, gekennzeichnet durch einen Gehalt an mindestens einem substituierten Pyridazinon der Formel (I).

7. Verfahren zur Bekämpfung von tierischen Schädlingen, dadurch gekennzeichnet, daß man substituierte Pyridazinone der Formel (I) auf tierische Schädlinge und/oder ihren Lebensraum einwirken läßt.

8. Verwendung von substituierten Pyridazinonen der Formel (I) zur Bekämpfung von tierischen Schädlingen.

9. Verfahren zur Herstellung von Schädlingsbekämpfungsmitteln, dadurch gekennzeichnet, daß man substituierte Pyridazinone der Formel (I) mit Streckmitteln und/oder oberflächenaktiven Mitteln vermischt.

10. Alkohole der Formel (V a)

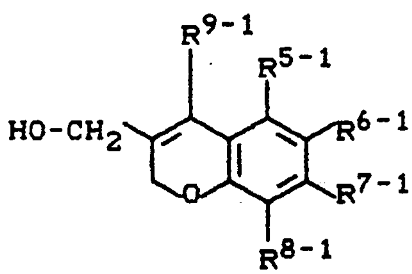

(V a)

in welcher
$R^{5-1}$, $R^{6-1}$, $R^{7-1}$, $R^{8-1}$ und $R^{9-1}$ unabhängig voneineinander jeweils für Wasserstoff, für Halogen wie vorzugsweise Fluor, Chlor, Brom, für jeweils geradkettiges oder verzweigtes Alkyl, Alkoxy oder Alkylthio mit jeweils 1 bis 4 Kohlenstoffatomen wie vorzugsweise Methyl, Ethyl, n-, i-, s- oder t-Butyl, Methoxy, Ethoxy oder Methylthio sowie für jeweils geradkettiges oder verzweigtes Halogenalkyl, Halogenalkoxy oder Halogenalkylthio mit jeweils 1 bis 9 gleichen oder verschiedenen Halogenatomen, wie vorzugsweise Trifluormethyl, Trifluormethoxy, Difluormethoxy oder Trifluormethylthio stehen, wobei jedoch nicht gleichzeitig alle Substituenten für Wasserstoff stehen.